Europäisches Patentamt

European Patent Office

Office européen des brevets

(11) Publication number: **0 412 641 A2**

# EUROPEAN PATENT APPLICATION

(21) Application number: 90306685.0

(51) Int. Cl.⁵: **A61K 31/59**

(22) Date of filing: **19.06.90**

The title of the invention has been amended (Guidelines for Examination in the EPO, A-III, 7.3).

(30) Priority: **22.06.89 JP 158411/89**

(43) Date of publication of application:
**13.02.91 Bulletin 91/07**

(84) Designated Contracting States:
**AT BE CH DE DK ES FR GB IT LI NL SE**

(71) Applicant: **TEIJIN LIMITED**
**6-7, Minamihonmachi 1-chome Chuo-ku**
**Osaka-shi Osaka 541(JP)**

(72) Inventor: **Mano, Tomiya**
**1-23-17, Kitakasugaoka**
**Ibaraki-shi, Osaka(JP)**
Inventor: **Ishizuka, Seiichi**
**3-18-4, Tamadaira**
**Hino-shi, Tokyo(JP)**
Inventor: **Uotani, Yasuhiro**
**2-92-1, Shinmei-cho**
**Koshigaya-shi, Saitama(JP)**

(74) Representative: **Votier, Sidney David et al**
**CARPMAELS & RANSFORD 43, Bloomsbury**
**Square**
**London WC1A 2RA(GB)**

(54) **Use of vitamin D in retinopathy.**

(57) An eyeground disease therapeutic treatment composition containing, as an active ingredient, an active vitamin D such as $1\alpha$-hydroxyvitamin D, $1\alpha,24$-dihydroxyvitamin D, $1\alpha,25$-dihydroxyvitamin D, $24,25$-dihydroxyvitamin D, $1\alpha,24,25$-trihydroxyvitamin D, $24,24$-$F_2$-$1\alpha,25$-dihydroxyvitamin D, $26,26,26,27,27,27$-$F_6$-$1\alpha,25$-dihydroxyvitamin D, $22$-oxa-$1\alpha,25$-dihydroxyvitamin D, $1\alpha,24(R)$-dihydroxy-22-dehydrovitamin D-26,27-cyclopropane and $1\alpha,24(S)$-dihydroxy-22-dehydrovitamin D-26,27-cyclopropane.

# EYEGROUND DISEASE THERAPEUTIC TREATMENT COMPOSITION

## BACKGROUND OF THE INVENTION

### 1. Field of the Invention

The present invention relates to an eyeground disease therapeutic treatment composition containing an active vitamin D as an active ingredient.

### 2. Description of the Related Art

The gliacytes of the retina are cells equivalent to the gliacytes of the brain (astrocytes, etc.), and these cells have the functions of supporting nervous tissues and supplying nutrients under normal conditions.

In various eyeground diseases such as diabetic retinopathy and retinal detachment, an abnormal growth of the gliacytes occurs, resulting in conditions which can be treated only with great difficulty, and sometimes leading to a loss of sight.

As the method of therapeutic treatment of eyeground disease caused by such an abnormal growth of the gliacytes of the retina, currently the therapy of a surgical excision of the growth on the retina is practiced, but this growth cannot be completely removed and thus frequently will recur. For a drug therapy, experiments have been carried out using steroids, 5-FU agents, and corhitine, but an effective drug is not clinically available.

As mentioned above, in various eyeground diseases, a differentiation-growth abnormality of the gliacytes is observed.

## SUMMARY OF THE INVENTION

Accordingly, the objects of the present invention are to eliminate the above-mentioned disadvantages of the prior art and to provide an effective eyeground therapeutic agent.

Other objects and advantages of the present invention will be apparent from the following description.

In accordance with the present invention, there is provided an eyeground disease therapeutic treatment composition comprising an active vitamin D as the active ingredient.

## DESCRIPTION OF THE PREFERRED EMBODIMENTS

The active vitamin D's such as $1\alpha$-hydroxyvitamin D, $1\alpha,24$-dihydroxyvitamin D, and $1\alpha,25$-dihydroxyvitamin D, etc., as is well known in the art, promote the absorption and conveyance of calcium in the small intestine, and have actions such as regulating bone resorption and bone formation in bones, and thus are therapeutic agents which are more useful than vitamin D as a therapeutic agent for various diseases based on a calcium metabolism abnormality, and are indispensable to the therapy of diseases based on a vitamin D metabolism abnormality. Further, recently, the cell differentiation-induction actions thereof have been discovered and, for example, the actions thereof against psoriasis have been investigated. Accordingly, the present inventors made an investigation into the influence of such an active vitamin D on the growth of gliacytes, and surprisingly found that a very small amount of an active vitamin D has the action of inhibiting the growth of the gliacytes of the retina, to thereby accomplish the present invention.

The active vitamin D's usable in the present invention include, for example, active vitamin $D_2$, active vitamin $D_3$, and derivatives thereof. Specific examples thereof are those corresponding thereto, such as $1\alpha$-hydroxyvitamin D, $1\alpha,24$-dihydroxyvitamin D, $1\alpha,25$-dihydroxyvitamin D, $24,25$-dihydroxyvitamin D, $1\alpha,24,25$-trihydroxyvitamin D, $24,24$-$F_2$-$1\alpha,25$-dihydroxyvitamin D, $26,26,26,27,27,27$-$F_6$-$1\alpha,25$-dihydroxyvitamin D, $22$-oxa-$1\alpha,25$-dihydroxyvitamin D, $1\alpha,24(R)$-dihydroxy-22-dehydrovitamin D-26,27-cyclopropane, and $1\alpha,24(S)$-dihydroxy-22-dehydrovitamin D-26,27-cyclopropane.

The eyeground disease referred to in the present invention is that caused by an abnormal growth of the retina glyacytes, as exemplified by, for example, diabetic retinopathy and retinal dispatchment.

These active ingredients can be used in the form of, for example, oral agents such as soft capsules, hard capsules, tablets, solutions, and syrups, and injections and eyedrops, formed by using appropriate excipients according to methods known in the art.

The active ingredients are usually used together with appropriate carriers, including excipients, according to conventional methods.

Examples of the carriers usable in the present invention are vegetable oil or mineral oils, white petrolatum, branched chain fats or oils, animal fats and high molecular weight alcohols for liquid agents or external application agents. Among these carriers, vegetable oils (corn oil, cotton oil, coconut oil, almond oils, especially middle chain length fatty acid triglycerides are preferred.

Examples of the carriers usable for solid agents are cellulose derivatives (crystalline cellulose, hydroxypropyl cellulose, hydroxypropyl-methyl cellulose, methyl cellulose), polyvinylpyrolidone, dextrin, cyclodextrin, casein, lactose, mannitol, gelatin or starch. Among these carriers, lactose and polyvinylpyrolidone, etc., are preferred. Although there are no limitations to the content of the active ingredient, i.e., the active vitamin D in the composition, the content of the active ingredient is preferably $(0.00004 - 0.2) \times 10^{-4}\%$ by weight, more preferably $(0.001 - 0.08) \times 10^{-4}\%$ by weight in the composition.

The dose of the active ingredient is generally about 0.01 to 10 $\mu$g/day/human, with administrations of generally once to 3 times/day, and the preparation is preferably prepared so as to satisfy such conditions.

The eyeground disease therapeutic treatment composition can be also used in combination with a therapeutic agent, for a drug therapeutic method.

## Examples

The present invention will now be further illustrated by, but is by no means limited to, the following Examples.

## Example 1

A growth medium is prepared by adding 20% of FBS (Fetal Bovine Serum) to a 50%/5% mixture of DMEM (Dulbecco's Modified Eagle Medium and F12 (Ham's F12 Medium) (basic medium).

Cultured retina gliacytes of a human being (prepared according to Investigative Ophthalmology & Visual Science, 28, (Suppl), 208, 1987) are suspended in 7 ml of the growth medium. A culture dish (35 mm, manufactured by Falcon) is then scored to produce 26 x 26 squares, and the growth medium having the cells suspended therein is added dropwise in portions of 0.2 ml per square. The next day, 1.5 ml of the growth medium is added, and two days (48 hours) after the dropwise addition of the cells, the cell number is counted. As a control, one cultured in the basic medium (no serum) is used. The cells are counted for 100 cells or more, the suspension is then washed once with DMEM, and thereafter, changed to an experimental liquid. As the experimental liquid, those shown in Table 1, i.e., those having the active vitamin D added at concentrations of 0 to 100 M to 20% FBS, were used. After 48 hours, the cell number at the same scored square was counted, to determine the growth rate. The results are shown in Table 1.

Table 1

| Kind of experimental liquid | Cell growth rate |
|---|---|
| 20% FBS (Control) | 1.42 (n = 6) |
| 20% FBS + D₃* 1 nM | 1.26 (n = 6) |
| " 10 nM | 1.27 (n = 6) |
| " 100 nM | 1.32 (n = 6) |

D₃*: 1α,25(OH)₂D₃

As clearly seen in Table 1, the active type vitamin D has an inhibitory effect on the growth of gliacytes.

## Example 2

When the experiments are carried out under the same conditions, except that FBS was not added to the basic medium as in the growth medium of Example 1, the same cell growth inhibitory action of $1\alpha,25$-$(OH)_2D_3$ was found. The results are shown in Table 2.

Table 2

| Kind of experimental liquid | Cell growth rate |
|---|---|
| 0% FBS (Control) | 1.34 (n = 6) |
| 0% FBS + D₃* 1 nM | 1.25 (n = 6) |
| " 10 nM | 1.18 (n = 6) |
| " 100 nM | 1.27 (n = 6) |

D₃*: $1\alpha,25(OH)_2D_3$

**Claims**

1. An eyeground disease therapeutic treatment composition comprising an active vitamin D, as an active ingredient, and a carrier therefore.

2. An eyeground therapeutic treatment composition as claimed in claim 1, wherein the active vitamin D is at least one compound selected from the group consisting of $1\alpha$-hydroxyvitamin D, $1\alpha,24$-dihydroxyvitamin D, $1\alpha,25$-dihydroxyvitamin D, $24,25$-dihydroxyvitamin D, $1\alpha,24,25$-trihydroxyvitamin D, $24,24$-$F_2$-$1\alpha,25$-dihydroxyvitamin D, $26,26,26,27,27,27$,-$F_6$-$1\alpha,25$-dihydroxyvitamin D, $22$-oxa-$1\alpha,25$-dihydroxyvitamin D, $1\alpha,24(R)$-dihydroxy-22-dehydrovitamin D-26,27-cyclopropane and $1\alpha,24(S)$-dihydroxy-22-dehydrovitamin D-26,27-cyclopropane.

3. An eyeground therapeutic treatment composition as claimed in claim 1, wherein the content of the active ingredient is $(0.00004 - 0.2) \times 10^{-4}$% by weight of the composition.